(19) Europäisches Patentamt
European Patent Office
Office européen des brevets

(11) **EP 0 577 972 B1**

(12) **EUROPÄISCHE PATENTSCHRIFT**

(45) Veröffentlichungstag und Bekanntmachung des Hinweises auf die Patenterteilung:
**24.01.1996 Patentblatt 1996/04**

(51) Int. Cl.$^6$: **C07C 29/10**, C07C 31/20, C07C 45/64, C07C 29/141, C07C 47/19

(21) Anmeldenummer: **93108905.6**

(22) Anmeldetag: **03.06.1993**

(54) **Verfahren zur Herstellung von 1,3-Propandiol**

Process for the preparation of 1,3-propanediol

Procédé pour la préparation de 1,3-propanediol

(84) Benannte Vertragsstaaten:
**AT BE CH DE DK ES FR GB IE IT LI NL PT SE**

(30) Priorität: **10.07.1992 DE 4222708**

(43) Veröffentlichungstag der Anmeldung:
**12.01.1994 Patentblatt 1994/02**

(73) Patentinhaber: **Degussa Aktiengesellschaft D-60311 Frankfurt (DE)**

(72) Erfinder:
• **Haas, Thomas, Dr.**
  **W-6000 Frankfurt/Main 1 (DE)**
• **Arntz, Dietrich, Dr.**
  **W-6370 Oberursel (DE)**

(56) Entgegenhaltungen:
**DE-C- 898 894        US-A- 4 806 658
US-A- 5 093 537**

**Beschreibung**

Die Erfindung richtet sich auf ein Verfahren zur Herstellung von 1,3-Propandiol (PD) auf der Basis der Hydratisierung von Acrolein zu 3-Hydroxypropionaldehyd (HPA) mit anschließender katalytischer Hydrierung. Erfindungsgemäß wird aus dem aus diesem Prozeß als Nebenprodukt entstehenden 4-Oxa-1,7-heptandiol (OD) gleichfalls 1,3-Propandiol gewonnen.

1,3-Propandiol besitzt als Monomerbaustein für Polyester und Polyurethane sowie als Ausgangsstoff für die Synthese cyclischer Verbindungen vielseitige Anwendungsmöglichkeiten.

Zur Herstellung von 1,3-Propandiol sind verschiedene Verfahren bekannt, die entweder von einem Molekülaufbau aus einem $C_2$- und $C_1$-Baustein oder vorzugsweise direkt von einem $C_3$-Baustein, wie insbesondere Acrolein, ausgehen. Die von Acrolein ausgehenden Verfahren zur Herstellung von 1,3-Propandiol basieren auf 2-Reaktionsstufen, nämlich (a) der Hydratisierung von Acrolein in Gegenwart eines sauren Hydratisierungskatalysators und (b) der katalytischen Hydrierung des 3-Hydroxypropionaldehyd enthaltenden, von nicht umgesetztem Acrolein befreiten Reaktionsgemischs der Stufe (a). Durch destillative Aufarbeitung des Reaktionsgemischs der Stufe (b), das außer 1,3-Propandiol auch Wasser und oberhalb 1,3-Propandiol siedende Nebenprodukte enthält, darunter 4-Oxa-1,7-heptandiol, wird reines 1,3-Propandiol gewonnen.

Die Formelgleichungen für die Stufen (a) und (b) lauten

$$CH_2=CH\text{-}CHO + H_2O \xrightarrow{\text{Kat}^{*)}} HOCH_2\text{-}CH_2\text{-}CHO \qquad \text{(a)}$$

$$HOCH_2\text{-}CH_2\text{-}CHO + H_2 \xrightarrow{\text{Kat}^{**)}} HOCH_2\text{-}CH_2\text{-}CH_2OH \qquad \text{(b)}$$

Wie aus der US-PS 2,434,110 bekannt ist, läßt sich die Hydratisierung bei erhöhter Temperatur unter Verwendung einer 5 bis 30 gew.-%igen Lösung von Acrolein in Wasser in Gegenwart einer Säure, wie z. B. Schwefelsäure, Phosphorsäure oder sauren Salzen dieser Säuren hydratisieren, wobei 3-Hydroxypropionaldehyd entsteht. Die Hydrierung des von nicht umgesetztem Acrolein befreiten Reaktionsgemischs läßt sich mit üblichen Hydrierkatalysatoren, welche ein oder mehrere hydrierwirksame Metalle wie z. B. Fe, Co, Ni, Cu, Ag, Mo, W, V, Cr, Rh, Pd, Os, Ir, Pt enthalten, durchführen. Die Ausbeute an 1,3-Propandiol wird maßgeblich von der Selektivität der Hydratisierungsstufe beeinflußt. Es wurden daher verschiedene Katalysatorsysteme vorgeschlagen, um die Hydratisierung mit höherer Selektivität im technischen Maßstab in einfacher Weise durchführen zu können. Beispielsweise wurden als Hydratisierungskatalysatoren Kationenaustauscherharze mit Phosphonsäuregruppen (DE-OS 39 26 136), chelatbildende Ionenaustauscher, wie solche mit Methyleniminodiessigsäure-Ankergruppen (DE-OS 40 38 192), Säure-Base-Puffer aus organischen Carbonsäuren oder Phosphorsäure und Salzen dieser Säuren, welche im Reaktionsgemisch zu einem pH-Wert von 2 bis 5 führen (DE-Patentanmeldung P 41 38 981.6) sowie anorganische Trägerstoffe mit basischen Aktivitätszentren, welche teilweise mit einer einwertigen Säure in durch Wasser nicht ablösbarer Form belegt sind (DE-Patentanmeldung P 41 38 982.4), vorgeschlagen. Üblicherweise wird zur Hydratisierung ein Acrolein-Wassergemisch im Gewichtsverhältnis von 1 zu 2 bis 1 zu 20 eingesetzt und die Umsetzung bei 30 bis 120 °C bei einem Druck im Bereich von 1 bis 20 bar batchweise oder kontinuierlich durchgeführt.

Die katalytische Hydrierung des vom Acrolein befreiten Reaktionsgemischs der Hydratisierungsstufe wird im allgemeinen bei einem pH-Wert im Bereich von 2,5 bis 6,0 und einer Temperatur im Bereich von 30 bis 180 °C durchgeführt. Zweckmäßigerweise wird die Hydrierung bis zu einem Umsatz im Bereich von 50 bis 95 % bei 30 bis 80 °C und die weitere Hydrierung bis zu 100 %igem Umsatz bei 100 bis 180 °C durchgeführt. Gemäß dem Verfahren der deutschen Patentanmeldung 41 32 663.5 sind Trägerkatalysatoren aus Titandioxid, auf dem Platin in feinverteilter Form vorliegt, besonders gute Hydrierkatalysatoren, mit welchen sowohl ein hoher Umsatz als auch eine hohe Selektivität erzielt werden.

Ein wesentlicher Nachteil aller bisher bekanntgewordenen Verfahren zur Herstellung von 1,3-Propandiol durch Hydratisierung von Acrolein und katalytische Hydrierung des 3-Hydroxypropionaldehyds (HPA) ist darin zu sehen, daß durch verschiedene Nebenreaktionen, insbesondere während der Hydratisierungsstufe, die Gesamtausbeute an 1,3-Propandiol gemindert wird. Bei der Aufarbeitung des Reaktionsgemisches aus der katalytischen Hydrierung konnten aus dem Hochsiederanteil (Siedepunkt oberhalb desjenigen von 1,3-Propandiol) als Hauptprodukte 4-Oxa-1,7-heptandiol und 4-Hydroxy-3-hydroxymethyl-tetrahydropyran nachgewiesen werden.

Aufgabe der vorliegenden Erfindung ist, einen Weg aufzuzeigen, die Ausbeute an 1,3-Propandiol im gattungsgemäßen Verfahren zur Herstellung von 1,3-Propandiol aus Acrolein in einfacher Weise zu erhöhen und den Anteil an zu entsorgenden hochsiedenden Nebenbestandteilen zu vermindern.

\*) = saurer Hydratisierungskatalysator
\*\*) = Hydrierkatalysator

Gefunden wurde ein Verfahren zur Herstellung von 1,3-Propandiol, umfassend die Stufen

(a) Hydratisierung von Acrolein in Gegenwart eines sauren Hydratisierungskatalysators,
(b) katalytische Hydrierung des 3-Hydroxypropionaldehyd enthaltenden, von nicht umgesetztem Acrolein befreiten Reaktionsgemischs der Stufe (a) und
(c) destillative Aufarbeitung des Wasser, 1,3-Propandiol und oberhalb 1,3-Propandiol siedende Nebenprodukte enthaltenden Reaktionsgemischs der Stufe (b),

welches dadurch gekennzeichnet ist, daß man aus den oberhalb 1,3-Propandiol siedenden Nebenprodukten 4-Oxa-1,7-heptandiol destillativ abtrennt, dieses 4-Oxa-1,7-heptandiol zwecks Etherspaltung in wäßriger Lösung bei 100 bis 300 °C mit einem sauren Feststoffkatalysator behandelt und das vom Feststoffkatalysator befreite Reaktionsgemisch in die Stufe (c) zurückführt.

Die vorbekannten Stufen (a) und (b), also die Hydratisierung und die katalytische Hydrierung, werden in an sich bekannter Weise, beispielsweise nach einem Verfahren der eingangs gewürdigten Dokumente durchgeführt. Erfindungswesentlich ist, daß bei der destillativen Aufarbeitung (c) des Reaktionsgemischs der Hydrierstufe nicht nur Wasser und anschließend 1,3-Propandiol aodestilliert werden, sondern daß aus dem Hochsiederanteil 4-Oxa-1,7-heptandiol abdestilliert und anschließend der Etherspaltung zugeführt wird. Sofern der gesamte Hochsiederanteil der Etherspaltung unterworfen wird, entstehen hierbei auch solche Nebenprodukte, darunter 3-Hydroxymethyl-tetrahydropyran, welche im Falle der Rückführung des gesamten Reaktionsgemischs aus der Etherspaltung in der destillativen Aufarbeitungsstufe (c) wegen ähnlicher Siedepunkte die Gewinnung eines reinen 1,3-Propandiols verhindern.

An sich kann 4-Oxa-1,7-heptandiol in an sich bekannter Weise in wäßriger Lösung in Gegenwart von Mineralsäuren zu 1,3-Propandiol gespalten werden. Eine derartige Maßnahme ist jedoch unzweckmäßig, weil das Reaktionsgemisch der Etherspaltung nicht in die destillative Aufarbeitungsstufe (c) zurückgeführt werden kann. Im Falle einer separaten Aufarbeitung des Reaktionsgemischs der Etherspaltung unter Verwendung von Mineralsäuren kommt es jedoch zu einem hohen Abwasseranteil.

Durch die Verwendung saurer Feststoffkatalysatoren zum Zwecke der Etherspaltung von 4-Oxa-1,7-heptandiol im erfindungsgemäßen Verfahren kann das vom Feststoffkatalysator befreite Reaktionsgemisch ohne jegliche weitere Aufarbeitungsmaßnahme in die destillative Aufarbeitungsstufe zurückgeführt werden.

Dies ist ein wesentlicher Vorteil des erfindungsgemäßen Verfahrens. Zur Etherspaltung des erfindungsgemäßen Verfahrens können unterschiedliche saure Feststoffkatalysatoren eingesetzt werden, beispielsweise gamma-Aluminiumoxid, saure Ionenaustauscher, natürliche und synthetische Zeolithe und mit Säuren in durch Wasser nicht ablösbarer Form belegte anorganische Träger, beispielsweise mit Phosphorsäure behandeltes und anschließend kalziniertes Titandioxid (siehe DE-Patentanmeldung P 41 38 982.4). Aufgrund der mit den verschiedenen sauren Feststoffkatalysatoren erhältlichen, sehr unterschiedlichen Umsätze und Selektivitäten werden als Feststoffkatalysator saure Zeolithe mit einem Si/Al-Atomverhältnis von größer 2 bis etwa 100, insbesondere größer 10 bis 40, bevorzugt. Besonders geeignet sind Zeolithe vom Typ ZSM5. Dealuminierte Zeolithe vom Y-Typ katalysieren gleichfalls die Etherspaltung, weisen aber nach bisherigen Erkenntnissen eine geringere Langzeitstabilität auf als ZSM5-Zeolithe.

Obgleich die erfindungsgemäße Etherspaltung von 4-Oxa-1,7-heptandiol in verdünnter oder konzentrierter wäßriger Lösung möglich ist, werden Lösungen mit einem Gehalt zwischen 5 und 40 Gew.-%, insbesondere 10 bis 30 Gew.-%, 4-Oxa-1,7-heptandiol bevorzugt eingesetzt. Durch die Reaktionstemperatur werden der Umsatz und die Selektivität der Etherspaltung beeinflußt. Vorzugsweise wird die Etherspaltung im Temperaturbereich zwischen 150 und 250 °C durchgeführt. Um Wasserverluste während der Etherspaltung zu vermeiden, wird im allgemeinen in geschlossenen Apparaturen unter dem sich bei der Reaktionstemperatur einstellenden oder höheren Druck gearbeitet.

Die das erfindungsgemäße Verfahren kennzeichnende Etherspaltung läßt sich batchweise oder kontinuierlich in üblichen Reaktoren, welche für Reaktionen in Gegenwart von Feststoffkatalysatoren geeignet sind, durchführen. Besonders zweckmäßig ist es, den Feststoffkatalysator in einem Festbettreaktor anzuordnen und die wäßrige 4-Oxa-1,7-heptandiol-Lösung bei der gewünschten Reaktionstemperatur mit einer solchen Raumgeschwindigkeit LHSV (liquid hourly space veloursity) über das Festbett zu leiten, daß der gewünschte Umsatz erzielt wird. Wie aus den nachfolgenden Beispielen hervorgeht, lassen sich problemlos Umsätze und Selektivitäten im Bereich von 60 bis etwa 75 % erzielen. Durch das erfindungsgemäße Verfahren ist es somit möglich, einen großen Anteil des bei der Hydratisierung und nachfolgenden katalytischen Hydrierung als Nebenprodukt gebildeten 4-Oxa-1,7-heptandiols in einfacher Weise in 1,3-Propandiol zu überführen; gleichzeitig wird der Anfall an zu entsorgenden hochsiedenden Nebenprodukten um den der Ausbeutesteigerung entsprechenden Anteil gemindert.

**Beispiele 1 bis 6**

Zur Ermittlung der Wirksamkeit von Festbettkatalysatoren zur Etherspaltung von 4-Oxa-1,7-heptandiol (OD) wird jeweils ein 10 ml Stahlfläschchen mit 7 ml Katalysator befüllt und soviel Wasser zugegeben, daß der Katalysator gerade bedeckt ist. Zusätzlich wird OD zugegeben, um die gewünschte OD-Konzentration in der wäßrigen Phase einzustellen.

Die Probe wird für eine bestimmte Reaktionsdauer im Heißluftofen bei der angegebenen Temperatur geschüttelt. Dann wird die Probe im Wasserstrahl abgeschreckt und analysiert (c = Konzentration; PD = 1,3-Propandiol; Index E bzw. P = Einsatz bzw. Produkt; U = Umsatz; S = Selektivität).

| Beispiel Nr. | Katalysator | $T_R$ (°C) | t (h) | $C_{OD,E}$ (Gew.%) | $C_{OD,P}$ (Gew.%) | $C_{PD,P}$ (Gew.%) | U (%) | S (%) |
|---|---|---|---|---|---|---|---|---|
| 1 | Amberlyst (Typ: .....) | 150 | 2 | 20,00 | 18,06 | 1,26 | 9,70 | 57 |
| 2 | Mordenit | 250 | 2 | 7,63 | 6,71 | 0,76 | 12,10 | 73 |
| 3 | Mordenit | 250 | 2 | 20,00 | 16,73 | 1,66 | 16,40 | 45 |
| 4 | gamma-$Al_2O_3$ | 250 | 2 | 7,63 | 7,06 | 0,39 | 7,50 | 60 |
| 5 | dealuminierter Y-Zeolith (Atomverhältnis Si/Al = ca. 100) | 250 | 2 | 20,00 | 5,32 | 12,02 | 73,40 | 72 |
| 6 | Zeolith ZSM5 (Atomverhältnis Si/Al=14) | 180 | 2 | 20,00 | 4,80 | 10,34 | 76,00 | 60 |

**Beispiel 7**

In einer Laborapparatur mit einem Festbettreaktor mit einem ZSM5-Zeolithen (Si/Al=14) als Katalysator wird die Hydrolyse von OD kontinuierlich über einen längeren Zeitraum durchgeführt. Umsatz und Selektivität werden durch Analyse der Produktlösung bestimmt. Die Apparatur besteht aus einer Vorlage für die OD-Lösung, einer HPLC-Pumpe zur Förderung, einem Heißluftofen, in dem eine Vorheizstrecke sowie das Reaktionsrohr (160 x 15 mm i.D.) installiert sind. Nach dem Reaktor wird die Flüssigkeit auf Raumtemperatur gekühlt. Die gesamte Apparatur wird auf einem Druck von 50 bar gehalten, um das Verdampfen des Wassers zu vermeiden. Die Produktlösung wird in regelmäßigen zeitlichen Abständen analysiert.

| OD-Startkonzentration | 20 Gew.-% |
|---|---|
| Reaktionstemperatur | 240 - 250 °C |
| Reaktionsdruck | 50 bar |
| LHSV | 0,5 $h^{-1}$ |

Die gesamte Versuchsdauer betrug 300 h. Der Umsatz betrug 60 ± 5 %. Die Temperatur wurde im Laufe des Versuches von 240 °C auf 250 °C angehoben, um den Umsatz konstant zu halten. Die Selektivität der Reaktion betrug 70 ± 3 %.

**Beispiel 8**

Gesamtverfahren mit und ohne Oxdiolhydrolyse

Es wurden 2 kg $H_2O$ mit 400 g Acrolein gemischt. Die Acroleinhydratisierung wurde dann in einem Reaktionsrohr, das mit Ionenaustauscher Lewatit TP 208 (H-Form) befüllt war, durchgeführt. Die Reaktionstemperatur betrug 45 °C; LHSV = 0,5 $h^{-1}$. Anschließend wurde das nicht umgesetzte Acrolein bei vermindertem Druck (350 mbar) von der wäßrigen HPA-Lösung abgetrennt.

Der Acroleinumsatz betrug 51 %, die Selektivität bezüglich HPA betrug 85 % (HPA-Konzentration = 10,4 % nach Acroleinabtrennung).

Die HPA-Lösung wurde in einem Hydrierautoklaven mit Begasungsrührer hydriert. Der $H_2$-Druck betrug 135 bar, die Reaktionstemperatur 60 °C; als Katalysator wurden 20 g Raney-Nickel eingesetzt. Die Ausbeute an 1,3-Propandiol (PD), bezogen auf eingesetzten 3-Hydroxypropionaldehyd (HPA), betrug 99,8 %.

2200 g der erhaltenen wäßrigen HPA-Lösung wurde in zwei gleiche Hälften geteilt. Von der ersten Hälfte wurde in einer Destillationskolonne bei 50 mbar Wasser abdestilliert. Es blieben 134,1 g PD und Hochsieder im Sumpf. Nach Erhöhung der Sumpftemperatur auf 134 °C wurden 115,1 g PD abdestilliert. Die PD-Ausbeute über alle Stufen betrug damit 83 %. Im Sumpf blieben 18,7 g Hochsieder. Aus dem Sumpf wurden bei 20 mbar und 155 °C 13,1 g OD abdestilliert. Diese 13,1 g wurden mit 52,4 g $H_2O$ versetzt und gemäß Beispiel 7 an einem ZSM5 Zeolithen umgesetzt. Die Produktlösung enthielt 6,2 % nicht umgesetztes OD und 9,6 % PD. Diese Lösung wurde mit der zweiten Hälfte der Hydrierlösung vereinigt. Nach dem Abtrennen des $H_2O$ wurden 120,4 g PD und 16,5 g OD aodestilliert.

Gegenüber dem Versuch ohne OD-Hydrolyse wurden also 4,6 % mehr PD isoliert. Da der nicht umgesetzte OD-Anteil (3,4 g) bei kontinuierlicher Fahrweise rezykliert und mit etwa 70 % Selektivität umgesetzt wird, wird die PD-Ausbeute um weitere 2,4 % erhöht, so daß eine Gesamtausbeuteerhöhung von 7 % resultiert.

**Patentansprüche**

1.  Verfahren zur Herstellung von 1,3-Propandiol, umfassend die Stufen

    (a) Hydratisierung von Acrolein in Gegenwart eines sauren Hydratisierungskatalysators,
    (b) katalytische Hydrierung des 3-Hydroxypropionaldehyd enthaltenden, von nicht umgesetztem Acrolein befreiten Reaktionsgemisch der Stufe (a) und
    (c) destillative Aufarbeitung des Wasser, 1,3-Propandiol und oberhalb 1,3-Propandiol siedende Nebenprodukte enthaltenden Reaktionsgemischs der Stufe (b),

    dadurch gekennzeichnet,
    daß man aus den oberhalb 1,3-Propandiol siedenden Nebenprodukten 4-Oxa-1,7-heptandiol destillativ abtrennt, dieses 4-Oxa-1,7-heptandiol zwecks Etherspaltung in wäßriger Lösung bei 100 bis 300 °C mit einem sauren Feststoffkatalysator behandelt und das vom Feststoffkatalysator befreite Reaktionsgemisch in die Stufe (c) zurückführt.

2. Verfahren nach Anspruch 1,
dadurch gekennzeichnet,
daß man 4-Oxa-1,7-heptandiol in Form einer 5 bis 40 gew.-%igen, vorzugsweise 10 bis 30 gew.-%igen, wäßrigen Lösung der Etherspaltung zuführt.

3. Verfahren nach Anspruch 1 oder 2,
dadurch gekennzeichnet,
daß man als sauren Feststoffkatalysator für die Etherspaltung einen sauren Zeolithen, vorzugsweise saure Zeolithe mit einem Si/Al-Atomverhältnis von größer 2 bis etwa 100, insbesondere größer 10 bis 40, verwendet.

4. Verfahren nach Anspruch 3,
dadurch gekennzeichnet,
daß man einen Zeolithen vom Typ ZSM5 verwendet.

5. Verfahren nach einem oder mehreren der Ansprüche 1
bis 4,
dadurch gekennzeichnet,
daß man die Etherspaltung bei 150 bis 250 °C durchführt.

6. Verfahren nach einem oder mehreren der Ansprüche 1 bis 5,
dadurch gekennzeichnet,
daß man die Etherspaltung kontinuierlich durchführt, indem die wäßrige Lösung von 4-Oxa-1,7-heptandiol über einen in einem Festbettreaktor angeordneten sauren Feststoffkatalysator leitet.

**Claims**

1. Process for the preparation of 1,3-propanediol comprising the stages

(a) hydration of acrolein in the presence of an acid hydration catalyst,
(b) catalytic hydrogenation of the reaction mixture from stage (a), which contains 3-hydroxypropionaldehyde and from which unreacted acrolein has been removed, and
(c) working-up, by distillation, of the reaction mixture from stage (b), which contains water, 1,3-propanediol and by-products having boiling points higher than that of 1,3-propanediol,

characterised in that 4-oxa-1,7-heptanediol is separated by distillation from the by-products having boiling points higher than that of 1,3-propanediol, and is treated in aqueous solution at from 100 to 300°C with an acid solid catalyst in order to split out ether, and the reaction mixture from which the solid catalyst has been removed is returned into stage (c).

2. Process according to Claim 1, characterised in that 4-oxa-1,7-heptanediol is fed to ether splitting in the form of a from 5 to 40 wt-%, preferably from 10 to 30 wt-%, aqueous solution.

3. Process according to Claim 1 or 2, characterised in that an acid zeolite, preferably acid zeolites having a Si/Al atomic ratio greater than from 2 to approximately 100, in particular greater than from 10 to 40, is used as an acid solid catalyst in order to split out ether.

4. Process according to Claim 3, characterised in that a zeolite of the ZSM5 type is used.

5. Process according to one or more of Claims 1 to 4, characterised in that ether is split out at from 150 to 250°C.

6. Process according to one or more of Claims 1 to 5, characterised in that ether is split out continuously by feeding the aqueous solution of 4-oxa-1,7-heptanediol over an acid solid catalyst disposed in a fixed bed reactor.

**Revendications**

1. Procédé de préparation de 1,3-propanediol comprenant les étapes de :

   (a) hydratation de l'acroléine en présence d'un catalyseur acide d'hydratation,
   (b) hydrogénation catalytique du mélange réactionnel de l'étape (a) libéré de l'acroléine qui n'a pas réagi contenant le 3-hydroxypropionaldéhyde et,
   (c) fractionnement de l'eau par distillation, du 1,3-propanediol et des sous-produits de point d'ébullition supérieur à celui du 1,3-propanediol contenus dans le mélange réactionnel de l'étape (b), caractérisé en ce qu'on sépare par distillation le sous-produit 4-oxa-1,7-heptanediol bouillant plus haut que le 1,3-propanediol, on traite ce 4-oxa-1,7-heptanediol pour couper l'éther dans une solution aqueuse de 100 à 300°C avec un catalyseur acide solide et on recycle le mélange réactionnel libéré du catalyseur solide à l'étape (c).

2. Procédé selon la revendication 1, caractérisé en ce qu'on soumet à la coupure de l'éther du 4-oxa-1,7-heptanediol sous forme d'une solution aqueuse de 5 à 40 % en poids, de préférence 10 à 30 % en poids.

3. Procédé selon la revendication 1 ou 2, caractérisé en ce qu'on utilise comme catalyseur acide solide pour la coupure de l'éther une zéolithe acide, de préférence une zéolithe acide avec un rapport atomique Si/Al supérieur à 2 jusqu'à environ 100, notamment supérieur à 10 jusqu'à 40.

4. Procédé selon la revendication 3, caractérisé en ce qu'on utilise une zéolithe du type ZSM5.

5. Procédé selon l'une ou plusieurs des revendications 1 à 4, caractérisé en ce qu'on réalise la coupure d'éther entre 150 et 250°C.

6. Procédé selon l'une ou plusieurs des revendications 1 à 5, caractérisé en ce qu'on réalise la coupure d'éther en continu en faisant passer la solution aqueuse de 4-oxa-1,7-heptanediol sur un catalyseur acide solide placé dans un réacteur à lit fixe.